# EUROPEAN PATENT APPLICATION

(11) **EP 4 786 608 A1**
(43) Date of publication of application: **05.08.2026**
(21) Application number: 24872849.5
(22) Date of filing: 24.09.2024
(51) Int. Cl.: C12Q 1/6883

(54) **EPIGENETIC METHYLATION MARKER FOR PREDICTING METABOLIC SYNDROME, AND KIT USING SAME**

(30) Priority: 25.09.2023 KR 20230127796; 25.09.2023 KR 20240009338; 25.09.2023 KR 20240009350; 25.09.2023 KR 20240009354
(71) Applicant: SCL Healthcare, Co., Ltd., Yongin-si Gyeonggi-do 16954 (KR)
(72) Inventor: LEE, Sanghoo, Gyeonggi-do 16954 (KR); HONG, Jeonghoon, Gyeonggi-do 16954 (KR); LEE, Eunhye, Gyeonggi-do 16954 (KR); BAIK, Sae Yun, Gyeonggi-do 16954 (KR); LEE, Mi-Kyeong, Gyeonggi-do 16954 (KR); LEE, Kyoung-Ryul, Gyeonggi-do 16954 (KR)
(74) Representative: Latscha Schöllhorn Partner AG
(86) International application number: PCT/KR2024/014347
(87) International publication number: WO 2025/071141

(57) **Abstract**

The present invention relates to an epigenetic methylation marker for predicting metabolic syndrome, and a kit for predicting metabolic syndrome using same. Enabling specific prediction of disease risk rates for each condition within metabolic syndrome, the biomarker of the present invention is expected to be actively applicable in the medical and healthcare fields.

## Description

### [Technical Field]

The present invention relates to an epigenetic methylation marker for predicting metabolic syndrome, and kit using same. The kit for predicting metabolic syndrome may comprise an agent for measuring the methylation level of the epigenetic methylation marker for predicting metabolic syndrome presented in the present invention.

### [Background Art]

Recently, as studies based on Next Generation Sequencing (NGS) have been making remarkable progress, it has been revealed that the expression patterns of genetic information contained in DNA inherited from parents can be differentially regulated by various acquired factors. This is referred to as epigenetics, and major factors known as mechanisms of epigenetics include the action of tissue-specific transcription factors, DNA methylation, histone modification, the action of non-coding RNA, and signals from outside the cell. In addition, it has been continuously reported that epigenetic variations caused by these major factors are associated with the occurrence of various diseases including cancer. In particular, DNA methylation in the human genome is an important clue in epigenetics and is dynamically regulated during development. Since these phenomena are essential for studies on intracellular biological processes and gene regulation, it is expected that the possibility of treatment of human diseases can be expanded based on an understanding of the structure and function of major factors of epigenetic mechanisms.

Meanwhile, metabolic syndrome, which is referred to as a disease of modern people, means a cluster of metabolic abnormal states such as hyperglycemia (fasting), hypertension, hypertriglyceridemia, low level of HDL cholesterol, and an increase in waist circumference due to central obesity, and metabolic syndrome is known to increase the risk of developing metabolic diseases such as cardiovascular disease and diabetes. When three or more diseases are present among the five risk factors, it is diagnosed as metabolic syndrome. Therefore, metabolic syndrome is not a single disease, but a comprehensive disease that occurs due to a complex association of genetic predisposition and environmental factors.

In modern times, the prevalence of metabolic syndrome has increased explosively and has become a problem, and the prevalence of metabolic syndrome in adults worldwide is 20 to 25%, 35% in the United States, and up to 30% in Korea. Such metabolic syndrome may cause serious complications if left untreated for a long time; however, there is still no technology capable of accurately predicting metabolic syndrome.

Accordingly, the present invention has been devised to solve the above problems, and relates to a biomarker for predicting metabolic syndrome. Since the biomarker of the present invention enables specific prediction of the risk rate of onset for each disease of metabolic syndrome, it is expected to be actively used in the fields of medicine and public health.

### [Disclosure]

### [Technical Problem]

The present invention has been devised to solve the problems of the prior art as described above, and relates to an epigenetic methylation marker as a biomarker for predicting metabolic syndrome.

In one aspect, the present invention provides a method for predicting or diagnosing the onset of metabolic syndrome.

In another aspect, the present invention provides a composition for predicting or diagnosing the onset of metabolic syndrome.

In still another aspect, the present invention provides a kit for predicting or diagnosing the onset of metabolic syndrome.

In still another aspect, the present invention provides an apparatus for predicting or diagnosing the onset of metabolic syndrome.

However, the technical problems to be achieved by the present invention are not limited to the problems mentioned above, and other problems not mentioned will be clearly understood by those skilled in the art from the following description.

### [Technical Solution]

Hereinafter, various embodiments described herein will be described with reference to figures. In the following description, numerous specific details are set forth, such as specific configurations, compositions, and processes, etc., in order to provide a thorough understanding of the present disclosure. However, certain embodiments may be practiced without one or more of these specific details, or in combination with other known methods and configurations. In other instances, known processes and preparation techniques have not been described in particular detail in order to not unnecessarily obscure the present disclosure. Reference throughout this specification to "one embodiment" or "an embodiment" means that a particular feature, configuration, composition, or characteristic described in connection with the embodiment is included in at least one embodiment of the present disclosure. Thus, the appearances of the phrase "in one embodiment" or "an embodiment" in various places throughout this specification are not necessarily referring to the same embodiment of the present disclosure. Additionally, the particular features, configurations, compositions, or characteristics may be combined in any suitable manner in one or more embodiments.

Unless otherwise stated in the specification, all the scientific and technical terms used in the specification have the same meanings as commonly understood by those skilled in the technical field to which the present disclosure pertains.

Throughout the specification, when a part is described as "comprising" a certain component, it means that it may further include other components rather than excluding other components unless specifically stated otherwise.

In this specification, the term "metabolic syndrome" means that an individual has three or more of five risk factors (hypertension, hyperglycemia, hypertriglyceridemia, low high-density lipoprotein cholesterol, and central obesity) that increase the risk of health problems including heart disease, diabetes, and stroke, and in relation to the metabolic syndrome, hyperglycemia, hypertension, hyperlipidemia, obesity, and cardiovascular and cerebrovascular diseases are explosively increasing. Since such metabolic syndrome may cause serious complications if left untreated for a long time, it is most important not only to diagnose early, but also to predict whether an individual belongs to a group at high risk of developing metabolic syndrome before onset. However, the development of a technology for predicting metabolic syndrome using epigenetics is still insufficient.

The epigenetics refers to the transmission of acquired specific traits to offspring regardless of gene base sequences, and means a change in gene function that occurs without a change in the DNA base sequence, that is, without gene mutation. The specific traits are gene expression patterns established as cells differentiate in each cell type, and occur by the combined action of tissue-specific transcription factor action, DNA methylation, histone modification, and extracellular signals. In particular, DNA methylation is a representative epigenetic change that directly changes gene function without a change in the gene code, can be transmitted to daughter cells, and is known to be associated with gene expression suppression and tumorigenesis.

Accordingly, the present invention relates to an epigenetic marker exhibiting high predictability for metabolic syndrome and specific detailed diseases constituting the metabolic syndrome.

According to one embodiment of the present invention, the present invention provides a composition for predicting or diagnosing metabolic syndrome comprising an agent for measuring the methylation level of a CpG site of one or more genes selected from the group consisting of *FAM20C* (Family with sequence similarity 20, member C), *PDGFA* (Platelet-derived growth factor subunit A), *TPD52L3* (Tumor Protein D52 Like 3), *PAX5* (Paired box 5), *SARDH* (Sarcosine Dehydrogenase), *MAD1L1* (Mitotic Arrest Deficient 1 Like 1), *CUX1* (Cut Like Homeobox 1), *IFRD1* (Interferon Related Developmental Regulator 1), *PTPRN2* (Protein Tyrosine Phosphatase Receptor Type N2), *NOXA1* (NADPH Oxidase Activator 1), *FAM102A* ((Myc-DDK-tagged)-Human family with sequence similarity 102, member A), *NACC2* (NACC Family Member 2), *NUDT1* (Nudix Hydrolase 1), and *SNX8* (Sorting Nexin 8). At this time, the CpG site may be one or more CpG sites selected from the group consisting of chr9:6328440, chr9:36949608, chr9:136545908, chr9:136549044, chr7:1880699, chr7:1880700, chr7:1881369, chr7:1881370, chr7:1885069, chr7:1885070, chr7:1885114, chr7:1885115, chr7:1885119, chr7:1885221, chr7:1886937, chr7:1886938, chr7:1887361, chr7:1887362, chr7:1889520, chr7:1889521, chr7:1890925, chr7:1890926, chr7:1910374, chr7:1910450, chr7:1912056, chr7:1912057, chr7:1914757, chr7:1914758, chr7:1923695, chr7:1923696, chr7:1938655, chr7:1938656, chr7:1939765, chr7:1939766, chr7:1948712, chr7:1948713, chr7:1948755, chr7:1948756, chr7:1973579, chr7:1973580, chr7:1976457, chr7:1976862, chr7:1977906, chr7:1989946, chr7:2012996, chr7:2012997, chr7:2019875, chr7:2020994, chr7:2020995, chr7:2027323, chr7:2027324, chr7:2027354, chr7:2027355, chr7:2046830, chr7:2046831, chr7:2050401, chr7:2050402, chr7:2082536, chr7:2172066, chr7:2195443, chr7:101917140, chr7:157448562, chr7:157455211, chr7:157455296, chr9:140322640, chr9:130703789, chr9:138911484, chr7:2289889, chr7:2298227, chr7:2314651, chr7:2314652, chr7:220974, chr7:220975, chr7:223592, chr7:545084, chr7:1865921, chr7:1865922, chr7:1880737, chr7:1881265, chr7:1881266, chr7:1948948, chr7:1950615, chr7:1968227, chr7:2017445, chr7:2017446, chr7:2060127, chr7:2082524, chr7:2106607, chr7:2121196, and chr7:112087033, and the metabolic syndrome may include one or more selected from the group consisting of hyperglycemia, central obesity, hypertension, and non-alcoholic fatty liver disease.

As a more preferred embodiment, when the metabolic syndrome includes hyperglycemia, the CpG site may include one or more CpG sites selected from the group consisting of chr9:6328440, chr9:36949608, chr9:136545908, chr9:136549044, chr7:220974, chr7:220975, chr7:223592, and chr7:545084, and when the metabolic syndrome includes central obesity, the CpG site may include one or more CpG sites selected from the group consisting of chr7:1880699, chr7:1880700, chr7:1881369, chr7:1881370, chr7:1885069, chr7:1885070, chr7:1885114, chr7:1885115, chr7:1885119, chr7:1885221, chr7:1886937, chr7:1886938, chr7:1887361, chr7:1887362, chr7:1889520, chr7:1889521, chr7:1890925, chr7:1890926, chr7:1910374, chr7:1910450, chr7:1912056, chr7:1912057, chr7:1914757, chr7:1914758, chr7:1923695, chr7:1923696, chr7:1938655, chr7:1938656, chr7:1939765, chr7:1939766, chr7:1948712, chr7:1948713, chr7:1948755, chr7:1948756, chr7:1973579, chr7:1973580, chr7:1976457, chr7:1976862, chr7:1977906, chr7:1989946, chr7:2012996, chr7:2012997, chr7:2019875, chr7:2020994, chr7:2020995, chr7:2027323, chr7:2027324, chr7:2027354, chr7:2027355, chr7:2046830, chr7:2046831, chr7:2050401, chr7:2050402, chr7:2082536, chr7:2172066, chr7:2195443, chr7:101917140, chr7:157448562, chr7:157455211, chr7:157455296, chr9:140322640, chr9:130703789, chr9:138911484, chr7:1865921, chr7:1865922, chr7:1880737, chr7:1881265, chr7:1881266, chr7:1948948, chr7:1950615, chr7:1968227, chr7:2017445, chr7:2017446, chr7:2060127, chr7:2082524, chr7:2106607, chr7:2121196, and chr7:112087033, and when the metabolic syndrome includes hypertension, the CpG site may include chr7:2289889, and when the metabolic syndrome includes non-alcoholic fatty liver disease, the CpG site may include one or more CpG sites selected from the group consisting of chr7:2298227, chr7:2314651, and chr7:2314652, but is not limited thereto.

In the composition for predicting or diagnosing metabolic syndrome of the present invention, the agent for measuring the methylation level of the CpG site of the gene may include one or more selected from the group consisting of bisulfite or a salt thereof, a methylation-sensitive restriction enzyme, a primer specific for a methylated sequence at the CpG site of the promoter of the gene, a primer specific for an unmethylated sequence, a methylated CpG binding domain, and an antibody that specifically binds to methylcytosine, and the methylation-sensitive restriction enzyme may include one or more selected from the group consisting of SmaI, SacII, EagI, HpaII, MspI, BssHII, BstUI, and NotI.

In another embodiment of the present invention, the present invention provides a kit for predicting or diagnosing metabolic syndrome comprising an agent for measuring the methylation level of a CpG site of one or more genes selected from the group consisting of *FAM20C* (Family with sequence similarity 20, member C), *PDGFA* (Platelet-derived growth factor subunit A), *TPD52L3* (Tumor Protein D52 Like 3), *PAX5* (Paired box 5), *SARDH* (Sarcosine Dehydrogenase), *MAD1L1* (Mitotic Arrest Deficient 1 Like 1), *CUX1* (Cut Like Homeobox 1), *IFRD1* (Interferon Related Developmental Regulator 1), *PTPRN2* (Protein Tyrosine Phosphatase Receptor Type N2), *NOXA1* (NADPH Oxidase Activator 1), *FAM102A* ((Myc-DDK-tagged)-Human family with sequence similarity 102, member A), *NACC2* (NACC Family Member 2), *NUDT1* (Nudix Hydrolase 1), and *SNX8* (Sorting Nexin 8). At this time, the kit preferably includes the above-described composition for predicting or diagnosing metabolic syndrome. Accordingly, descriptions of specific diseases within metabolic syndrome and CpG sites matched thereto are omitted in order to prevent excessive complexity of the specification. In addition, the kit of the present invention may be a qRT-PCR kit, a PCR kit, a methylation-specific PCR kit, a real-time methylation-specific PCR kit, a PCR kit using a methylation DNA-specific binding protein, a DNA microarray kit, a MALDITOFF kit, a MassARRAY kit, a MethyLight kit, a QAMA kit, an ERMA kit, a HeavyMethyl kit, a QBSUPT kit, an MS-SNuPE kit, a MethylQuant kit, a Quantitative PCR sequencing kit, and an oligonucleotide-based microarray kit, and any kit commonly used in the art may be used without limitation.

In another embodiment of the present invention, the present invention provides a method for providing information for predicting or diagnosing metabolic syndrome, comprising a step of measuring the methylation level of a CpG site of one or more genes selected from the group consisting of *FAM20C* (Family with sequence similarity 20, member C), *PDGFA* (Platelet-derived growth factor subunit A), *TPD52L3* (Tumor Protein D52 Like 3), *PAX5* (Paired box 5), *SARDH* (Sarcosine Dehydrogenase), *MAD1L1* (Mitotic Arrest Deficient 1 Like 1), *CUX1* (Cut Like Homeobox 1), *IFRD1* (Interferon Related Developmental Regulator 1), *PTPRN2* (Protein Tyrosine Phosphatase Receptor Type N2), *NOXA1* (NADPH Oxidase Activator 1), *FAM102A* ((Myc-DDK-tagged)-Human family with sequence similarity 102, member A), *NACC2* (NACC Family Member 2), *NUDT1* (Nudix Hydrolase 1), and *SNX8* (Sorting Nexin 8) from a sample isolated from a subject. At this time, the CpG site may be one or more CpG sites selected from the group consisting of chr9:6328440, chr9:36949608, chr9:136545908, chr9:136549044, chr7:1880699, chr7:1880700, chr7:1881369, chr7:1881370, chr7:1885069, chr7:1885070, chr7:1885114, chr7:1885115, chr7:1885119, chr7:1885221, chr7:1886937, chr7:1886938, chr7:1887361, chr7:1887362, chr7:1889520, chr7:1889521, chr7:1890925, chr7:1890926, chr7:1910374, chr7:1910450, chr7:1912056, chr7:1912057, chr7:1914757, chr7:1914758, chr7:1923695, chr7:1923696, chr7:1938655, chr7:1938656, chr7:1939765, chr7:1939766, chr7:1948712, chr7:1948713, chr7:1948755, chr7:1948756, chr7:1973579, chr7:1973580, chr7:1976457, chr7:1976862, chr7:1977906, chr7:1989946, chr7:2012996, chr7:2012997, chr7:2019875, chr7:2020994, chr7:2020995, chr7:2027323, chr7:2027324, chr7:2027354, chr7:2027355, chr7:2046830, chr7:2046831, chr7:2050401, chr7:2050402, chr7:2082536, chr7:2172066, chr7:2195443, chr7:101917140, chr7:157448562, chr7:157455211, chr7:157455296, chr9:140322640, chr9:130703789, chr9:138911484, chr7:2289889, chr7:2298227, chr7:2314651, chr7:2314652, chr7:220974, chr7:220975, chr7:223592, chr7:545084, chr7:1865921, chr7:1865922, chr7:1880737, chr7:1881265, chr7:1881266, chr7:1948948, chr7:1950615, chr7:1968227, chr7:2017445, chr7:2017446, chr7:2060127, chr7:2082524, chr7:2106607, chr7:2121196, and chr7:112087033, and the metabolic syndrome may include one or more selected from the group consisting of hyperglycemia, central obesity, hypertension, and non-alcoholic fatty liver disease. In addition, the method for providing information for predicting or diagnosing metabolic syndrome of the present invention may further comprise a step of predicting that a subject has a high risk of developing metabolic syndrome or diagnosing that the subject has already developed metabolic syndrome when one or more CpG sites selected from the group consisting of chr9:6328440, chr9:36949608, chr9:136545908, chr9:136549044, chr7:1880699, chr7:1880700, chr7:1881369, chr7:1881370, chr7:1885069, chr7:1885070, chr7:1885114, chr7:1885115, chr7:1885119, chr7:1885221, chr7:1886937, chr7:1886938, chr7:1887361, chr7:1887362, chr7:1889520, chr7:1889521, chr7:1890925, chr7:1890926, chr7:1910374, chr7:1910450, chr7:1912056, chr7:1912057, chr7:1914757, chr7:1914758, chr7:1923695, chr7:1923696, chr7:1938655, chr7:1938656, chr7:1939765, chr7:1939766, chr7:1948712, chr7:1948713, chr7:1948755, chr7:1948756, chr7:1973579, chr7:1973580, chr7:1976457, chr7:1976862, chr7:1977906, chr7:1989946, chr7:2012996, chr7:2012997, chr7:2019875, chr7:2020994, chr7:2020995, chr7:2027323, chr7:2027324, chr7:2027354, chr7:2027355, chr7:2046830, chr7:2046831, chr7:2050401, chr7:2050402, chr7:2082536, chr7:2172066, chr7:2195443, chr7:101917140, chr7:157448562, chr7:157455211, chr7:157455296, chr9:140322640, chr9:130703789, chr9:138911484, chr7:2289889, chr7:2298227, chr7:2314651, and chr7:2314652 are hypermethylated, or when one or more CpG sites selected from the group consisting of chr7:220974, chr7:220975, chr7:223592, chr7:545084, chr7:1865921, chr7:1865922, chr7:1880737, chr7:1881265, chr7:1881266, chr7:1948948, chr7:1950615, chr7:1968227, chr7:2017445, chr7:2017446, chr7:2060127, chr7:2082524, chr7:2106607, chr7:2121196, and chr7:112087033 are hypomethylated.

As a more preferred embodiment, when the metabolic syndrome comprises hyperglycemia, the method may comprise measuring the methylation level of CpG sites including one or more CpG sites selected from the group consisting of chr9:6328440, chr9:36949608, chr9:136545908, chr9:136549044, chr7:220974, chr7:220975, chr7:223592, and chr7:545084, and may further comprise predicting that a subject has a high risk of developing hyperglycemia or diagnosing that the subject has already developed hyperglycemia when one or more CpG sites selected from the group consisting of chr9:6328440, chr9:36949608, chr9:136545908, and chr9:136549044 are hypermethylated, or when one or more CpG sites selected from the group consisting of chr7:220974, chr7:220975, chr7:223592, and chr7:545084 are hypomethylated. When the metabolic syndrome comprises central obesity, the method may comprise measuring the methylation level of CpG sites including one or more CpG sites selected from the group consisting of chr7:1880699, chr7:1880700, chr7:1881369, chr7:1881370, chr7:1885069, chr7:1885070, chr7:1885114, chr7:1885115, chr7:1885119, chr7:1885221, chr7:1886937, chr7:1886938, chr7:1887361, chr7:1887362, chr7:1889520, chr7:1889521, chr7:1890925, chr7:1890926, chr7:1910374, chr7:1910450, chr7:1912056, chr7:1912057, chr7:1914757, chr7:1914758, chr7:1923695, chr7:1923696, chr7:1938655, chr7:1938656, chr7:1939765, chr7:1939766, chr7:1948712, chr7:1948713, chr7:1948755, chr7:1948756, chr7:1973579, chr7:1973580, chr7:1976457, chr7:1976862, chr7:1977906, chr7:1989946, chr7:2012996, chr7:2012997, chr7:2019875, chr7:2020994, chr7:2020995, chr7:2027323, chr7:2027324, chr7:2027354, chr7:2027355, chr7:2046830, chr7:2046831, chr7:2050401, chr7:2050402, chr7:2082536, chr7:2172066, chr7:2195443, chr7:101917140, chr7:157448562, chr7:157455211, chr7:157455296, chr9:140322640, chr9:130703789, chr9:138911484, chr7:1865921, chr7:1865922, chr7:1880737, chr7:1881265, chr7:1881266, chr7:1948948, chr7:1950615, chr7:1968227, chr7:2017445, chr7:2017446, chr7:2060127, chr7:2082524, chr7:2106607, chr7:2121196, and chr7:112087033, and may further comprise predicting that a subject has a high risk of developing central obesity or diagnosing that the subject has already developed central obesity when one or more CpG sites selected from the group consisting of chr7:1880699, chr7:1880700, chr7:1881369, chr7:1881370, chr7:1885069, chr7:1885070, chr7:1885114, chr7:1885115, chr7:1885119, chr7:1885221, chr7:1886937, chr7:1886938, chr7:1887361, chr7:1887362, chr7:1889520, chr7:1889521, chr7:1890925, chr7:1890926, chr7:1910374, chr7:1910450, chr7:1912056, chr7:1912057, chr7:1914757, chr7:1914758, chr7:1923695, chr7:1923696, chr7:1938655, chr7:1938656, chr7:1939765, chr7:1939766, chr7:1948712, chr7:1948713, chr7:1948755, chr7:1948756, chr7:1973579, chr7:1973580, chr7:1976457, chr7:1976862, chr7:1977906, chr7:1989946, chr7:2012996, chr7:2012997, chr7:2019875, chr7:2020994, chr7:2020995, chr7:2027323, chr7:2027324, chr7:2027354, chr7:2027355, chr7:2046830, chr7:2046831, chr7:2050401, chr7:2050402, chr7:2082536, chr7:2172066, chr7:2195443, chr7:101917140, chr7:157448562, chr7:157455211, chr7:157455296, chr9:140322640, chr9:130703789, and chr9:138911484 are hypermethylated, or when one or more CpG sites selected from the group consisting of chr7:1865921, chr7:1865922, chr7:1880737, chr7:1881265, chr7:1881266, chr7:1948948, chr7:1950615, chr7:1968227, chr7:2017445, chr7:2017446, chr7:2060127, chr7:2082524, chr7:2106607, chr7:2121196, and chr7:112087033 are hypomethylated. When the metabolic syndrome comprises hypertension, the method may comprise measuring the methylation level of a CpG site including chr7:2289889, and may further comprise predicting that a subject has a high risk of developing hypertension or diagnosing that the subject has already developed hypertension when the region chr7:2289889 is hypermethylated. Alternatively, when the metabolic syndrome comprises non-alcoholic fatty liver disease, the method may comprise measuring the methylation level of CpG sites including one or more CpG sites selected from the group consisting of chr7:2298227, chr7:2314651, and chr7:2314652, and may further comprise predicting that a subject has a high risk of developing non-alcoholic fatty liver disease or diagnosing that the subject has already developed non-alcoholic fatty liver disease when one or more CpG sites selected from the group consisting of chr7:2298227, chr7:2314651, and chr7:2314652 are hypermethylated. In this case, the subject refers to a target for predicting a risk of developing metabolic syndrome or diagnosing the onset thereof, and may include, without limitation, humans as well as animals susceptible to metabolic syndrome, such as dogs, horses, cattle, rats, goats, and rabbits. Further, the sample isolated from the subject may be a biological sample capable of detecting the presence or level of methylation, and may be DNA or nucleic acid extracted from the aforementioned tissues. In particular, any nucleic acid capable of detecting the presence of CpG at a methylated CpG site may be used, and is not limited thereto, and any nucleic acid in a purified or unpurified form may be used, including any nucleic acid comprising or suspected of comprising a nucleic acid sequence containing a target site (e.g., a CpG-containing nucleic acid). Specifically, the nucleic acid used for detecting the methylation status of a CpG site may be DNA, particularly genomic DNA, but is not limited thereto.

In the method for providing information for predicting or diagnosing metabolic syndrome of the present invention, the agent for measuring the methylation level of the CpG site of the gene may include one or more selected from the group consisting of bisulfite or a salt thereof, a methylation-sensitive restriction enzyme, a primer specific for a methylated sequence at the CpG site of the promoter of the gene, a primer specific for an unmethylated sequence, a methylated CpG binding domain, and an antibody that specifically binds to methylcytosine, and the methylation-sensitive restriction enzyme may include one or more selected from the group consisting of SmaI, SacII, EagI, HpaII, MspI, BssHII, BstUI, and NotI.

More specifically, when the method for providing information for predicting or diagnosing metabolic syndrome of the present invention is described step by step, the step of measuring the methylation level of a CpG site may comprise: (a) treating genomic DNA in an isolated sample with bisulfite or a salt thereof, or a methylation-sensitive restriction enzyme; and (b) amplifying the treated DNA by PCR using a primer capable of amplifying a target CpG site. At this time, various amplification methods may be used for measuring the methylation level of the CpG site. For example, it may be performed through PCR (Polymerase Chain Reaction), methylation-specific PCR, real time methylation specific PCR (real time methylation specific PCR), PCR using a methylation DNA-specific binding protein, DNA microarray, pyrosequencing, and bisulfite sequencing. As amplification methods other than PCR, ligase chain reaction (LCR), transcription amplification, selective amplification methods of target sequences, PCR using conserved sequences, PCR using random primers, nucleic acid sequence-based amplification (NASBA), and nick translation amplification methods may be used, but are not limited thereto. Other methods for measuring the methylation level include MALDITOFF, MassARRAY, MethyLight, QAMA (Quantitative analysis of ethylated alleles), ERMA (enzymatic regional methylation assay), HeavyMethyl, QBSUPT, MS-SNuPE, MethylQuant, Quantitative PCR sequencing, and oligonucleotide-based microarrays, but are not limited thereto. Probes, primers, and the like used for detecting the methylation state act through hybridization with a target nucleic acid. Hybridization conditions may be determined depending on specific experimental purposes and characteristics of nucleic acids used in the reaction. For example, the length of nucleic acids at the hybridization site, the degree of homology, nucleotide sequence composition (e.g., GC/AT composition ratio), and nucleic acid type (e.g., RNA, DNA) are considered in selecting hybridization conditions. In general, optimal conditions for increasing specificity vary depending on the hybridization reaction and may be determined through experiments. PCR primers for detecting the methylation level of CpG sites may be designed in various ways depending on the detection method. For example, in bisulfite analysis, COBRA (Combined Bisulfite Restriction Analysis), or Ms-SNuPE (Methylation-sensitive single nucleotide primer extension) analysis, the primers themselves are designed not to cover or hybridize to DNA methylation sites and potential sites, and sequence variations at sites where methylation occurs differentially are positioned between a pair of primers. Alternatively, the primers may be designed to specifically hybridize to methylated or unmethylated sites after chemical treatment, and if binding complementarity is sufficient so as not to interfere with hybridization, they may be designed to include additional sequences such as restriction enzyme sites, ligand binding sites, linkers, or repeat sequences. In addition, probes capable of specifically binding to a specific product may be used for detecting modified or unmodified DNA, and such probes may directly bind to a modified product or bind to an amplified product of a modified product, and may be labeled with various known materials for detection, for example, fluorescent materials, radioactive materials, bioluminescent materials, luminescent materials, chemiluminescent materials, enzymes, receptors, or ligands. Labeling methods are techniques widely known in the art and may be performed by conventional methods. Alternatively, the methylation level of CpG sites may be measured through BSAS (Bisulfite Amplicon Sequencing). The BSAS may be performed by various methods known in the art, for example, methylation may be measured by labeling methylated DNA with a fluorescent dye and hybridizing the labeled DNA to a DNA chip on which complementary probes are immobilized.

In another embodiment of the present invention, the present invention provides an apparatus for predicting or diagnosing metabolic syndrome comprising: a detection unit for measuring the methylation level of a CpG site of one or more genes selected from the group consisting of *FAM20C* (Family with sequence similarity 20, member C), *PDGFA* (Platelet-derived growth factor subunit A), *TPD52L3* (Tumor Protein D52 Like 3), *PAX5* (Paired box 5), *SARDH* (Sarcosine Dehydrogenase), *MAD1L1* (Mitotic Arrest Deficient 1 Like 1), *CUX1* (Cut Like Homeobox 1), *IFRD1* (Interferon Related Developmental Regulator 1), *PTPRN2* (Protein Tyrosine Phosphatase Receptor Type N2), *NOXA1* (NADPH Oxidase Activator 1), *FAM102A* ((Myc-DDK-tagged)-Human family with sequence similarity 102, member A), *NACC2* (NACC Family Member 2), *NUDT1* (Nudix Hydrolase 1), and *SNX8* (Sorting Nexin 8); and a determination unit for determining whether the CpG site is hypomethylated or hypermethylated. At this time, the apparatus may be configured such that the detection unit is operated according to the method for providing information for predicting or diagnosing metabolic syndrome of the present invention using the composition for predicting or diagnosing metabolic syndrome of the present invention. Accordingly, descriptions of specific diseases within metabolic syndrome, CpG sites corresponding thereto, and determination methods based on high/low expression of CpG sites in combination with specific diseases within metabolic syndrome are omitted in order to prevent excessive complexity of the specification.

In another embodiment of the present invention, the present invention provides a method for predicting or diagnosing metabolic syndrome comprising a step of measuring the methylation level of a CpG site of one or more genes selected from the group consisting of *FAM20C* (Family with sequence similarity 20, member C), *PDGFA* (Platelet-derived growth factor subunit A), *TPD52L3* (Tumor Protein D52 Like 3), *PAX5* (Paired box 5), *SARDH* (Sarcosine Dehydrogenase), *MAD1L1* (Mitotic Arrest Deficient 1 Like 1), *CUX1* (Cut Like Homeobox 1), *IFRD1* (Interferon Related Developmental Regulator 1), *PTPRN2* (Protein Tyrosine Phosphatase Receptor Type N2), *NOXA1* (NADPH Oxidase Activator 1), *FAM102A* ((Myc-DDK-tagged)-Human family with sequence similarity 102, member A), *NACC2* (NACC Family Member 2), *NUDT1* (Nudix Hydrolase 1), and *SNX8* (Sorting Nexin 8) from a sample isolated from a subject suspected of having metabolic syndrome. In the method, descriptions of specific diseases within metabolic syndrome, CpG sites corresponding thereto, and determination methods based on high/low expression of CpG sites in combination with specific diseases within metabolic syndrome are the same as those defined in the above-described method for providing information for predicting or diagnosing metabolic syndrome, and thus are omitted in order to prevent excessive complexity of the specification.

In another embodiment of the present invention, the present invention provides a use of an agent capable of measuring the methylation level of a CpG site of one or more genes selected from the group consisting of *FAM20C* (Family with sequence similarity 20, member C), *PDGFA* (Platelet-derived growth factor subunit A), *TPD52L3* (Tumor Protein D52 Like 3), *PAX5* (Paired box 5), *SARDH* (Sarcosine Dehydrogenase), *MAD1L1* (Mitotic Arrest Deficient 1 Like 1), *CUX1* (Cut Like Homeobox 1), *IFRD1* (Interferon Related Developmental Regulator 1), *PTPRN2* (Protein Tyrosine Phosphatase Receptor Type N2), *NOXA1* (NADPH Oxidase Activator 1), *FAM102A* ((Myc-DDK-tagged)-Human family with sequence similarity 102, member A), *NACC2* (NACC Family Member 2), *NUDT1* (Nudix Hydrolase 1), and *SNX8* (Sorting Nexin 8) for predicting or diagnosing metabolic syndrome. In the use, descriptions of specific diseases within metabolic syndrome, CpG sites corresponding thereto, and determination methods based on high/low expression of CpG sites in combination with specific diseases within metabolic syndrome are the same as those defined in the above-described method for providing information for predicting or diagnosing metabolic syndrome, and thus are omitted in order to prevent excessive complexity of the specification.

### [Advantageous Effects]

When the epigenetic methylation marker of the present invention is used, it is possible to predict the likelihood of onset of metabolic syndrome or diagnose the onset through analysis of the correlation between DNA methylation and expression values even with a small amount of sample, and thus it is possible to facilitate personalized treatment and management of patients with metabolic syndrome by predicting metabolic syndrome or a risk thereof.

### [Description of Drawings]

Fig. 1 is a schematic diagram showing an analysis process for confirming a methylation state between a metabolic disease group and a normal control group according to one embodiment of the present invention.
Fig. 2 is a result of comparing methylation patterns in chromosome 7 regions between a metabolic disease group and a normal control group according to one embodiment of the present invention.
Fig. 3 is a result of comparing methylation patterns in chromosome 9 regions between a metabolic disease group and a normal control group according to one embodiment of the present invention.

### [Best Mode]

As a result of confirming the methylation status of genomic DNA extracted from blood (3 cc of whole blood) of a metabolic disease group or a normal group using NEBNext^{®} Enzymatic Methyl-seq (EM-seq^{™}; TWIST Bioscience), which is an enzymatic treatment method, DNA methylation hotspots specific to particular metabolic diseases were identified in chromosome 7 and chromosome 9. Specifically, metabolic syndrome was associated with methylation of *FAM20C*, *PDGFA*, *TPD52L3*, *PAX5*, *SARDH*, *MAD1L1*, *CUX1*, *IFRD1*, *PTPRN2*, *NOXA1*, *FAM102A*, *NACC2*, *NUDT1*, or *SNX8* genes.

### [Mode for Invention]

Hereinafter, the present invention will be described in more detail through examples. These examples are provided only for the purpose of describing the present invention more specifically, and it will be apparent to those skilled in the art that the scope of the present invention is not limited by these examples in accordance with the gist of the present invention.

Throughout the specification, when a part is described as "comprising" a certain component, it means that it may further include other components rather than excluding other components unless specifically stated otherwise.

### [Research Methods]

### 1. Detection of methylation.

As a conventional method for confirming DNA methylation, there has been a method of identifying methylated cytosine residues by chemically converting unmethylated cytosine into uracil through bisulfite treatment. When this method is used, after PCR, unmethylated cytosine is read as thymine in the nucleotide sequence, and methylated cytosine is read as cytosine. However, bisulfite treatment often causes DNA breakage, resulting in failure of methylation detection.

Therefore, in the present invention, an enzymatic treatment method, NEBNext^{®} Enzymatic Methyl-seq (EM-seq^{™}; TWIST Bioscience), was used to obtain results identical to those of the conventional method without a chemical conversion process by bisulfite. Ultimately, results with much higher reliability than those of the conventional method were obtained.

### 2. DNA methylation analysis.

### 2-1. DNA methylation

After preparing 200 ng of genomic DNA extracted from blood (3 cc of whole blood), fragmentation was performed using Qsonica so that an average size of 240±290 bp was obtained. The sample DNA was subjected to an end prep process in which dA-tailing was added, and then an adapter was ligated by an enzyme method. The adapter-ligated DNA was reacted with TET2 to oxidize 5mC and 5hmC regions, and at this time, an Fe(II) solution was used. After reacting for 1 hour, impurities were removed using beads. The oxidized DNA library was denatured using 0.1N NaOH, and since unmethylated cytosine is converted to thymine, cytosine was deaminated using an APOBEC enzyme to protect 5mC and 5hmC regions. Thereafter, impurities were removed using beads, and the purified deaminated library was amplified by PCR.

### 2-2. Amplification of methylated DNA

The DNA library prepared in the previous step was mixed with a methylation panel, dried at room temperature, and hybridized. The hybridization was performed by reacting for 2 hours at 60°C in a state in which the hybridization material and DNA were well mixed. The sample subjected to the hybridization reaction was rapidly and carefully washed using beads and a high-temperature (65°C) washing solution to remove the hybridization material. At this time, care was taken so that the temperature of the sample subjected to the hybridization reaction did not fall below 60°C. The washed sample was homogenized with beads using clean water, and PCR amplification was performed in the homogenized state, followed by removal of impurities to measure the concentration of the final library. The final library was sequenced using an Illumina NGS (NextSeq 550) system, and the average yield was confirmed to be 12 Gbp, and the average on-target rate was 41.6x.

### 3. Analysis of methylation pattern

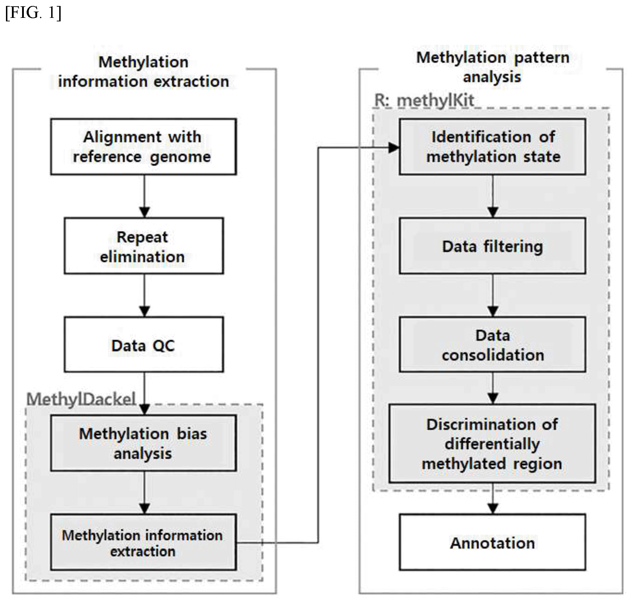

An analysis process for confirming the methylation state is shown in Fig. 1. First, the quality of the produced sequence was evaluated and low-quality sequence regions were removed (FastQC v0.11.9), and then methylated/unmethylated cytosine sequences were identified and mapped to a reference sequence (bwameth v0.2.4). Among the mapped reads, duplicate reads produced with identical sequences were present, so these were removed and the quality control (QC) of the data was confirmed (Picard v2.26.10). At this time, the depth of the target region was calculated using samtool (v1.14). Since bias may occur at both ends of reads when methylation has occurred, a process of checking this is required, and the positions and levels of methylation were analyzed using the methyldackel (v0.6.1) analysis tool.

Meanwhile, methylation information extracted from the metabolic disease group and the normal control group was analyzed as a difference in patterns between the two groups. Specifically, methylation call information was confirmed using the methylKit package (v1.24.0) included in the R program (v4.1.2), regions with depth < 10 were removed, and methylation call regions of the samples to be analyzed were merged to analyze similarity of patterns. The methylation patterns between the two groups were compared using the Pheatmap package included in the R program.

### [Research Results]

### 1. Comparison of clinical data between metabolic disease group and normal control group

Basic clinical data for a metabolic disease group (n=20) and a control group (n=20) classified as normal are shown in Table 1. The two groups showed large differences in systolic blood pressure, blood glucose, and triglycerides between the groups (p=2.2 ×10⁻⁶, p=1.3× 10⁻⁵, p=2.5×10⁻⁶), and also showed statistically significant differences in age, body weight, waist circumference, BMI, diastolic blood pressure, HDL, LDL, and γ-GTP (p<0.05). Hereinafter, comparison of methylation patterns between groups of the present invention was performed with reference to the above clinical data. However, no differences were observed between the two groups in height, total cholesterol, aspartate aminotransferase, alanine aminotransferase, creatinine, and glomerular filtration rate.

**[Table 1]**

| **clinical indicator** | | **control** | **metabolic syndrome** | ***P* value** |
|---|---|---|---|---|
| No. of study participants | | 20 | 20 | |
| | Age (years) | 42.0±9.9 | 54.5±11.1 | 6.0×10⁻⁴ |
| | Height (cm) | 160.3±5.4 | 157.7±6.2 | 0.1593 |
| | Weight (kg) | 56.5±6.8 | 62.9±10.8 | 3.0×10⁻² |
| | Waist measurement (cm) | 73.5±6.2 | 82.3±9.0 | 3.0×10⁻⁴ |
| | BMI (kg/m²) | 22.1±3.1 | 25.2±3.1 | 2.5×10⁻³ |
| | SBP (mmHg) | 110.0±8.9 | 127.7±11.1 | 2.2×10⁻⁶ |
| | DBP (mmHg) | 70.3±8.4 | 76.2±8.5 | 3.5×10⁻² |
| | Blood glucose (mg/dL) | 87.3±6.5 | 104.5±13.9 | 1.3×10⁻⁵ |
| | Total cholesterol (mg/dL) | 194.0±19.6 | 210.4±31.0 | 0.0526 |
| | HDL cholesterol (mg/dL) | 76.7±16.5 | 57.1±19.0 | 1.2×10⁻³ |
| | LDL cholesterol (mg/dL) | 103.4±20.2 | 123.4±33.6 | 2.8×10⁻³ |
| | Triglyceride (mg/dL) | 77.6±28.8 | 156.3±56.7 | 2.5×10⁻⁶ |
| | AST (U/L) | 21.7±8.8 | 22.8±4.7 | 0.6399 |
| | ALT (U/L) | 18.0±11.0 | 21.9±8.9 | 0.2232 |
| | γ-GTP (U/L) | 15.6±8.8 | 27.0±18.4 | 1.6×10⁻² |
| | Serum creatinine (mg/dL) | 0.7±0.1 | 0.7±0.1 | 0.2432 |
| | GFR (mL/min/1.73 m²) | 96.7±21.7 | 85.6±18.0 | 0.1654 |
| Values are shown as mean ± SD. | | | | |
| BMI, body mass index; SBP, systolic blood pressure; DBP, diastolic blood pressure; HDL-C, high-density lipoprotein-cholesterol; LDL-C, low-density lipoprotein cholesterol; AST, aspartate aminotransferase; ALT, alanine aminotransferase; γ-GTP, γ-glutamyltranspeptidase; GFR, glomerular filtration rate. | | | | |

### 2. Comparison of methylation patterns between metabolic disease group and normal control group

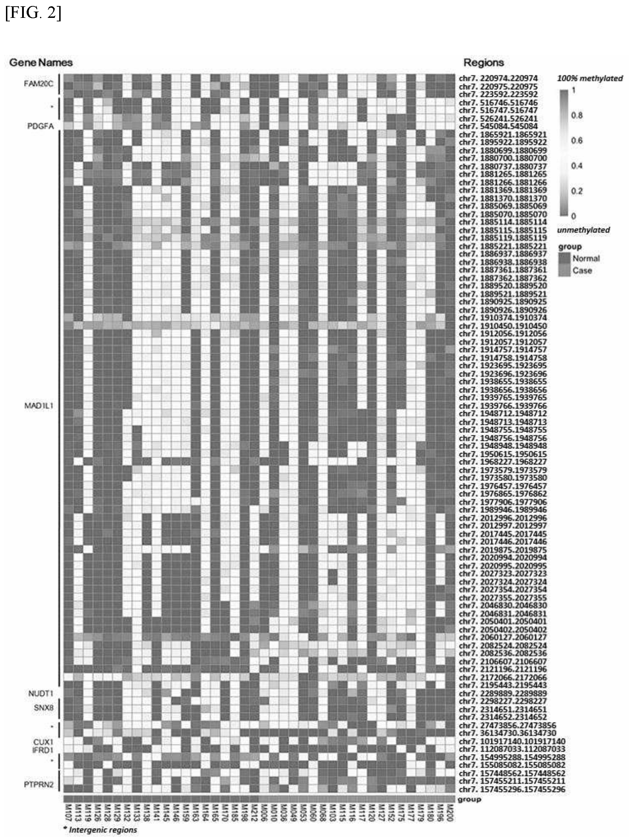

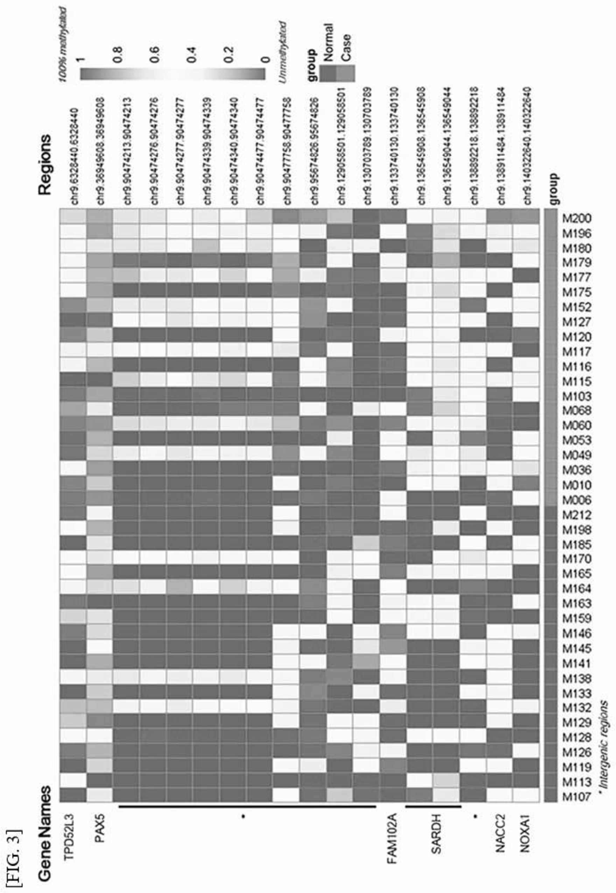

All samples including the metabolic disease group and the normal control group were analyzed for methylation status relative to a reference sequence, and using this information, methylation patterns between the metabolic disease group and the normal control group were comparatively analyzed. Statistical significance was calculated as P values using Fisher's exact test or logistic regression. The P values were corrected by applying Bonferroni correction, and Q values were calculated using a sliding linear model (SLIM). From this, regions having an adjusted P value < 5 × 10⁻⁸ and showing an increase of 25% or more in differential methylation (hypermethylation) or a decrease of 25% or more (hypomethylation) were extracted, and 464 regions showing methylation differences were examined using a heatmap. At this time, regions showing an increase of 25% or more in methylation relative to the reference sequence for all samples were indicated in red (hypermethylation), and regions showing a decrease of 25% or more were indicated in blue (hypomethylation). As a result, clear methylation differences between the normal control group and the metabolic disease group were identified particularly in chromosome 7 and chromosome 9 regions among the entire autosomal regions. The results of comparison of methylation patterns in chromosome 7 are shown in Fig. 2, and the results of comparison of methylation patterns in chromosome 9 are shown in Fig. 3, and DNA methylation hotspots specific to particular metabolic diseases in chromosome 7 and chromosome 9 are shown in Tables 2 to 5. In Tables 2 to 5, P values were adjusted by Bonferroni correction, Hyper-M indicates Hypermethylation, and Hypo-M indicates Hypomethylation.

**[Table 2]**

| **DNA methylation hotspots specific to hyperglycemia** | | | | | | | | |
|---|---|---|---|---|---|---|---|---|
| **Genes** | **CpG id** | **Chr** | **Position _start** | **Position _end** | **function s** | **Differential methylatio n (M)** | **P value** | **Q value** |
| *FAM20C* | | chr 7 | 220974 | 220974 | intronic | Hypo-M | 1.24E-14 | 1.24E-08 |
| *FAM20C* | | chr 7 | 220975 | 220975 | intronic | Hypo-M | 2.41E-13 | 2.41E-07 |
| *FAM20C* | cg1189615 1 | chr 7 | 223592 | 223592 | intronic | Hypo-M | 1.58E-48 | 1.58E-42 |
| *PDGFA* | | chr 7 | 545084 | 545084 | intronic | Hypo-M | 9.18E-17 | 9.18E-11 |
| *TPD52L* 3 | | chr 9 | 6328440 | 6328440 | exonic | Hyper-M | 2.05E-25 | 2.05E-19 |
| *PAX5* | | chr 9 | 36949608 | 36949608 | intronic | Hyper-M | 2.27E-15 | 2.27E-09 |
| *SARDH* | | chr 9 | 13654590 8 | 13654590 8 | intronic | Hyper-M | 6.26E-55 | 6.26E-49 |
| *SARDH* | | chr 9 | 13654904 4 | 13654904 4 | intronic | Hyper-M | 1.40E-24 | 1.40E-18 |

**[Table 3]**

| **DNA methylation hotspots specific to central obesity** | | | | | | | | |
|---|---|---|---|---|---|---|---|---|
| **Genes** | **CpG id** | **Chr** | **Position _start** | **Position _end** | **function s** | **Differential methylatio n (M)** | **P value** | **Q value** |
| *MAD1L1* | | chr 7 | 1865921 | 1865921 | intronic | Hypo-M | 1.01E-19 | 1.01E-13 |
| *MAD1L1* | | chr 7 | 1865922 | 1865922 | intronic | Hypo-M | 3.04E-24 | 3.04E-18 |
| *MAD1L1* | | chr 7 | 1880699 | 1880699 | intronic | Hyper-M | 6.27E-42 | 6.27E-36 |
| *MAD1L1* | | chr 7 | 1880700 | 1880700 | intronic | Hyper-M | 2.93E-33 | 2.93E-27 |
| *MAD1L1* | | chr 7 | 1880737 | 1880737 | intronic | Hypo-M | 1.06E-26 | 1.06E-20 |
| *MAD1L1* | | chr 7 | 1881265 | 1881265 | intronic | Hypo-M | 2.79E-47 | 2.79E-41 |
| *MAD1L1* | | chr 7 | 1881266 | 1881266 | intronic | Hypo-M | 7.04E-35 | 7.04E-29 |
| *MAD1L1* | | chr 7 | 1881369 | 1881369 | intronic | Hyper-M | 7.00E-39 | 7.00E-33 |
| *MAD1L1* | | chr 7 | 1881370 | 1881370 | intronic | Hyper-M | 6.34E-33 | 6.34E-27 |
| *MAD1L1* | | chr 7 | 1885069 | 1885069 | intronic | Hyper-M | 1.80E-40 | 1.80E-34 |
| *MAD1L1* | | chr 7 | 1885070 | 1885070 | intronic | Hyper-M | 6.63E-37 | 6.63E-31 |
| *MAD1L1* | | chr 7 | 1885114 | 1885114 | intronic | Hyper-M | 3.17E-33 | 3.17E-27 |
| *MAD1L1* | | chr 7 | 1885115 | 1885115 | intronic | Hyper-M | 1.75E-24 | 1.75E-18 |
| *MAD1L1* | | chr 7 | 1885119 | 1885119 | intronic | Hyper-M | 5.09E-32 | 5.09E-26 |
| *MAD1L1* | | chr 7 | 1885221 | 1885221 | intronic | Hyper-M | 6.89E-28 | 6.89E-22 |
| *MAD1L1* | | chr 7 | 1886937 | 1886937 | intronic | Hyper-M | 3.06E-27 | 3.06E-21 |
| *MAD1L1* | | chr 7 | 1886938 | 1886938 | intronic | Hyper-M | 2.61E-24 | 2.61E-18 |
| *MAD1L1* | | chr 7 | 1887361 | 1887361 | intronic | Hyper-M | 1.16E-35 | 1.16E-29 |
| *MAD1L1* | | chr 7 | 1887362 | 1887362 | intronic | Hyper-M | 9.26E-51 | 9.26E-45 |
| *MAD1L1* | | chr 7 | 1889520 | 1889520 | intronic | Hyper-M | 8.87E-30 | 8.87E-24 |
| *MAD1L1* | | chr 7 | 1889521 | 1889521 | intronic | Hyper-M | 2.20E-19 | 2.20E-13 |
| *MAD1L1* | | chr 7 | 1890925 | 1890925 | intronic | Hyper-M | 2.23E-50 | 2.23E-44 |
| *MAD1L1* | | chr 7 | 1890926 | 1890926 | intronic | Hyper-M | 2.79E-31 | 2.79E-25 |
| *MAD1L1* | | chr 7 | 1910374 | 1910374 | intronic | Hyper-M | 2.58E-58 | 2.58E-52 |
| *MAD1L1* | | chr 7 | 1910450 | 1910450 | intronic | Hyper-M | 5.20E-41 | 5.20E-35 |
| *MAD1L1* | | chr 7 | 1912056 | 1912056 | intronic | Hyper-M | 1.72E-31 | 1.72E-25 |
| *MAD1L1* | | chr 7 | 1912057 | 1912057 | intronic | Hyper-M | 1.12E-51 | 1.12E-45 |
| *MAD1L1* | | chr 7 | 1914757 | 1914757 | intronic | Hyper-M | 1.21E-16 | 1.21E-10 |
| *MAD1L1* | | chr 7 | 1914758 | 1914758 | intronic | Hyper-M | 4.73E-67 | 4.73E-61 |
| *MAD1L1* | cg1216970 0 | chr 7 | 1923695 | 1923695 | intronic | Hyper-M | 9.05E-22 | 9.05E-16 |
| *MAD1L1* | | chr 7 | 1923696 | 1923696 | intronic | Hyper-M | 3.13E-34 | 3.13E-28 |
| *MAD1L1* | | chr 7 | 1938655 | 1938655 | intronic | Hyper-M | 6.83E-27 | 6.83E-21 |
| *MAD1L1* | | chr 7 | 1938656 | 1938656 | intronic | Hyper-M | 3.96E-50 | 3.96E-44 |
| *MAD1L1* | | chr 7 | 1939765 | 1939765 | intronic | Hyper-M | 9.16E-27 | 9.16E-21 |
| *MAD1L1* | | chr 7 | 1939766 | 1939766 | intronic | Hyper-M | 1.62E-39 | 1.62E-33 |
| *MAD1L1* | | chr 7 | 1948712 | 1948712 | intronic | Hyper-M | 6.90E-38 | 6.90E-32 |
| *MAD1L1* | | chr 7 | 1948713 | 1948713 | intronic | Hyper-M | 2.33E-47 | 2.33E-41 |
| *MAD1L1* | cg1617827 1 | chr 7 | 1948755 | 1948755 | intronic | Hyper-M | 3.02E-40 | 3.02E-34 |
| *MAD1L1* | | chr 7 | 1948756 | 1948756 | intronic | Hyper-M | 1.58E-51 | 1.58E-45 |
| *MAD1L1* | | chr 7 | 1948948 | 1948948 | intronic | Hypo-M | 6.71E-24 | 6.71E-18 |
| *MAD1L1* | | chr 7 | 1950615 | 1950615 | intronic | Hypo-M | 3.89E-24 | 3.89E-18 |
| *MAD1L1* | | chr 7 | 1968227 | 1968227 | intronic | Hypo-M | 2.89E-62 | 2.89E-56 |
| *MAD1L1* | | chr 7 | 1973579 | 1973579 | intronic | Hyper-M | 1.16E-21 | 1.16E-15 |
| *MAD1L1* | | chr 7 | 1973580 | 1973580 | intronic | Hyper-M | 1.61E-29 | 1.61E-23 |
| *MAD1L1* | cg0307588 9 | chr 7 | 1976457 | 1976457 | exonic | Hyper-M | 3.72E-28 | 3.72E-22 |
| *MAD1L1* | | chr 7 | 1976862 | 1976862 | intronic | Hyper-M | 5.55E-27 | 5.55E-21 |
| *MAD1L1* | | chr 7 | 1977906 | 1977906 | intronic | Hyper-M | 2.08E-37 | 2.08E-31 |
| *MAD1L1* | | chr 7 | 1989946 | 1989946 | intronic | Hyper-M | 2.27E-18 | 2.27E-12 |
| *MAD1L1* | | chr 7 | 2012996 | 2012996 | intronic | Hyper-M | 2.04E-60 | 2.04E-54 |
| *MAD1L1* | | chr 7 | 2012997 | 2012997 | intronic | Hyper-M | 1.08E-47 | 1.08E-41 |
| *MAD1L1* | | chr 7 | 2017445 | 2017445 | intronic | Hypo-M | 1.62E-38 | 1.62E-32 |
| *MAD1L1* | | chr 7 | 2017446 | 2017446 | intronic | Hypo-M | 4.72E-53 | 4.72E-47 |
| *MAD1L1* | | chr 7 | 2019875 | 2019875 | intronic | Hyper-M | 1.01E-87 | 1.01E-81 |
| *MAD1L1* | | chr 7 | 2020994 | 2020994 | intronic | Hyper-M | 1.23E-73 | 1.23E-67 |
| *MAD1L1* | | chr 7 | 2020995 | 2020995 | intronic | Hyper-M | 4.60E-43 | 4.60E-37 |
| *MAD1L1* | | chr 7 | 2027323 | 2027323 | intronic | Hyper-M | 3.51E-57 | 3.51E-51 |
| *MAD1L1* | | chr 7 | 2027324 | 2027324 | intronic | Hyper-M | 2.11E-37 | 2.11E-31 |
| *MAD1L1* | | chr 7 | 2027354 | 2027354 | intronic | Hyper-M | 1.44E-63 | 1.44E-57 |
| *MAD1L1* | | chr 7 | 2027355 | 2027355 | intronic | Hyper-M | 6.32E-46 | 6.32E-40 |
| *MAD1L1* | | chr 7 | 2046830 | 2046830 | intronic | Hyper-M | 1.11E-37 | 1.11E-31 |
| *MAD1L1* | | chr 7 | 2046831 | 2046831 | intronic | Hyper-M | 1.05E-43 | 1.05E-37 |
| *MAD1L1* | cg2728661 4 | chr 7 | 2050401 | 2050401 | intronic | Hyper-M | 4.28E-52 | 4.28E-46 |
| *MAD1L1* | | chr 7 | 2050402 | 2050402 | intronic | Hyper-M | 6.04E-47 | 6.04E-41 |
| *MAD1L1* | | chr 7 | 2060127 | 2060127 | intronic | Hypo-M | 9.98E-39 | 9.98E-33 |
| *MAD1L1* | | chr 7 | 2082524 | 2082524 | intronic | Hypo-M | 7.58E-28 | 7.58E-22 |
| *MAD1L1* | | chr 7 | 2082536 | 2082536 | intronic | Hyper-M | 7.88E-36 | 7.88E-30 |
| *MAD1L1* | | chr 7 | 2106607 | 2106607 | intronic | Hypo-M | 1.13E-24 | 1.13E-18 |
| *MAD1L1* | | chr 7 | 2121196 | 2121196 | intronic | Hypo-M | 1.17E-58 | 1.17E-52 |
| *MAD1L1* | | chr 7 | 2172066 | 2172066 | intronic | Hyper-M | 6.64E-17 | 6.64E-11 |
| *MAD1L1* | | chr 7 | 2195443 | 2195443 | intronic | Hyper-M | 3.97E-23 | 3.97E-17 |
| *CUX1* | | chr 7 | 10191714 0 | 10191714 0 | intronic | Hyper-M | 1.28E-28 | 1.28E-22 |
| *IFRD1* | | chr 7 | 11208703 3 | 11208703 3 | intronic | Hypo-M | 3.67E-28 | 3.67E-22 |
| *PTPRN2* | | chr 7 | 15744856 2 | 15744856 2 | intronic | Hyper-M | 1.76E-23 | 1.76E-17 |
| *PTPRN2* | | chr 7 | 15745521 1 | 15745521 1 | intronic | Hyper-M | 6.48E-52 | 6.48E-46 |
| *PTPRN2* | | chr 7 | 15745529 6 | 15745529 6 | intronic | Hyper-M | 2.80E-25 | 2.80E-19 |
| *NOXA1* | | chr 9 | 14032264 0 | 14032264 0 | intronic | Hyper-M | 9.91E-42 | 9.91E-36 |
| *FAM102 A* | | chr 9 | 13070378 9 | 13070378 9 | exonic | Hyper-M | 9.46E-39 | 9.46E-33 |
| *NACC2* | | chr 9 | 13891148 4 | 13891148 4 | intronic | Hyper-M | 1.11E-31 | 1.11E-25 |

**[Table 4]**

| **DNA methylation hotspots specific to hypertension** | | | | | | | | |
|---|---|---|---|---|---|---|---|---|
| **Genes** | **CpG id** | **Chr** | **Position _start** | **Position _end** | **functions** | **Differential methylation (M)** | **P value** | **Q value** |
| *NUDT1* | | chr7 | 2289889 | 2289889 | intronic | Hyper-M | 5.64E-23 | 5.64E-17 |

**[Table 5]**

| **DNA methylation hotspots specific to non-alcoholic fatty liver disease** | | | | | | | | |
|---|---|---|---|---|---|---|---|---|
| **Genes** | **CpG id** | **Chr** | **Position _start** | **Position _end** | **functions** | **Differential methylation (M)** | **P value** | **Q value** |
| *SNX8* | | chr7 | 2298227 | 2298227 | intronic | Hyper-M | 5.27E-31 | 5.27E-25 |
| *SNX8* | | chr7 | 2314651 | 2314651 | intronic | Hyper-M | 1.71E-37 | 1.71E-31 |
| *SNX8* | | chr7 | 2314652 | 2314652 | intronic | Hyper-M | 1.05E-47 | 1.05E-41 |

Analysis of the above results showed that metabolic syndrome was associated with methylation of *FAM20C* (Family with sequence similarity 20, member C), *PDGFA* (Platelet-derived growth factor subunit A), *TPD52L3* (Tumor Protein D52 Like 3), *PAX5* (Paired box 5), *SARDH* (Sarcosine Dehydrogenase), *MAD1L1* (Mitotic Arrest Deficient 1 Like 1), *CUX1* (Cut Like Homeobox 1), *IFRD1* (Interferon Related Developmental Regulator 1), *PTPRN2* (Protein Tyrosine Phosphatase Receptor Type N2), *NOXA1* (NADPH Oxidase Activator 1), *FAM102A* ((Myc-DDK-tagged)-Human family with sequence similarity 102, member A), *NACC2* (NACC Family Member 2), *NUDT1* (Nudix Hydrolase 1), or *SNX8* (Sorting Nexin 8). In terms of specific DNA methylation hotspots, it was found that the risk of developing metabolic syndrome was high when one or more regions selected from the group consisting of chr9:6328440, chr9:36949608, chr9:136545908, chr9:136549044, chr7:1880699, chr7:1880700, chr7:1881369, chr7:1881370, chr7:1885069, chr7:1885070, chr7:1885114, chr7:1885115, chr7:1885119, chr7:1885221, chr7:1886937, chr7:1886938, chr7:1887361, chr7:1887362, chr7:1889520, chr7:1889521, chr7:1890925, chr7:1890926, chr7:1910374, chr7:1910450, chr7:1912056, chr7:1912057, chr7:1914757, chr7:1914758, chr7:1923695, chr7:1923696, chr7:1938655, chr7:1938656, chr7:1939765, chr7:1939766, chr7:1948712, chr7:1948713, chr7:1948755, chr7:1948756, chr7:1973579, chr7:1973580, chr7:1976457, chr7:1976862, chr7:1977906, chr7:1989946, chr7:2012996, chr7:2012997, chr7:2019875, chr7:2020994, chr7:2020995, chr7:2027323, chr7:2027324, chr7:2027354, chr7:2027355, chr7:2046830, chr7:2046831, chr7:2050401, chr7:2050402, chr7:2082536, chr7:2172066, chr7:2195443, chr7:101917140, chr7:157448562, chr7:157455211, chr7:157455296, chr9:140322640, chr9:130703789, chr9:138911484, chr7:2289889, chr7:2298227, chr7:2314651, and chr7:2314652 are hypermethylated, or when one or more regions selected from the group consisting of chr7:220974, chr7:220975, chr7:223592, chr7:545084, chr7:1865921, chr7:1865922, chr7:1880737, chr7:1881265, chr7:1881266, chr7:1948948, chr7:1950615, chr7:1968227, chr7:2017445, chr7:2017446, chr7:2060127, chr7:2082524, chr7:2106607, chr7:2121196, and chr7:112087033 are hypomethylated.

When analyzed by dividing into specific diseases within metabolic syndrome, hyperglycemia among metabolic syndrome was associated with methylation of *FAM20C*, *PDGFA*, *TPD52L3*, *PAX5*, or *SARDH* genes. In terms of specific DNA methylation hotspots, it was found that the risk of developing hyperglycemia was high when one or more regions selected from the group consisting of chr9:6328440, chr9:36949608, chr9:136545908, and chr9:136549044 are hypermethylated, or when one or more regions selected from the group consisting of chr7:220974, chr7:220975, chr7:223592, and chr7:545084 are hypomethylated.

Central obesity was associated with methylation of *MAD1L1*, *CUX1*, *IFRD1*, *PTPRN2*, *NOXA1*, *FAM102A*, or *NACC2* genes. In terms of specific DNA methylation hotspots, it was found that the risk of developing central obesity was high when one or more regions selected from the group consisting of chr7:1880699, chr7:1880700, chr7:1881369, chr7:1881370, chr7:1885069, chr7:1885070, chr7:1885114, chr7:1885115, chr7:1885119, chr7:1885221, chr7:1886937, chr7:1886938, chr7:1887361, chr7:1887362, chr7:1889520, chr7:1889521, chr7:1890925, chr7:1890926, chr7:1910374, chr7:1910450, chr7:1912056, chr7:1912057, chr7:1914757, chr7:1914758, chr7:1923695, chr7:1923696, chr7:1938655, chr7:1938656, chr7:1939765, chr7:1939766, chr7:1948712, chr7:1948713, chr7:1948755, chr7:1948756, chr7:1973579, chr7:1973580, chr7:1976457, chr7:1976862, chr7:1977906, chr7:1989946, chr7:2012996, chr7:2012997, chr7:2019875, chr7:2020994, chr7:2020995, chr7:2027323, chr7:2027324, chr7:2027354, chr7:2027355, chr7:2046830, chr7:2046831, chr7:2050401, chr7:2050402, chr7:2082536, chr7:2172066, chr7:2195443, chr7:101917140, chr7:157448562, chr7:157455211, chr7:157455296, chr9:140322640, chr9:130703789, and chr9:138911484 are hypermethylated, or when one or more regions selected from the group consisting of chr7:1865921, chr7:1865922, chr7:1880737, chr7:1881265, chr7:1881266, chr7:1948948, chr7:1950615, chr7:1968227, chr7:2017445, chr7:2017446, chr7:2060127, chr7:2082524, chr7:2106607, chr7:2121196, and chr7:112087033 are hypomethylated.

Hypertension was associated with methylation of the *NUDT1* gene. In terms of specific DNA methylation hotspots, it was found that the risk of developing hypertension was high when the region chr7:2289889 is hypermethylated.

Non-alcoholic fatty liver disease was associated with methylation of the *SNX8* gene. In terms of specific DNA methylation hotspots, it was found that the risk of developing non-alcoholic fatty liver disease was high when one or more regions selected from the group consisting of chr7:2298227, chr7:2314651, and chr7:2314652 are hypermethylated.

Therefore, by analyzing methylation patterns of the above DNA methylation hotspots, it is expected that prediction of the risk of developing metabolic syndrome, specifically hyperglycemia, central obesity, hypertension, or non-alcoholic fatty liver disease, and disease management will be possible.

Although the present disclosure has been described in detail with reference to the specific features, it will be apparent to those skilled in the art that this description is only of a preferred embodiment thereof, and does not limit the scope of the present disclosure. Thus, the substantial scope of the present disclosure will be defined by the appended claims and equivalents thereto.

### [Industrial Applicability]

Since the biomarker of the present invention enables specific prediction of the risk rate of onset for each disease of metabolic syndrome, it is expected to be actively used in the fields of medicine and public health.

## Claims

1. A composition for predicting or diagnosing metabolic syndrome, comprising an agent for measuring a methylation level of a CpG site of one or more genes selected from the group consisting of *FAM20C* (Family with sequence similarity 20, member C), *PDGFA* (Platelet-derived growth factor subunit A), *TPD52L3* (Tumor Protein D52 Like 3), *PAX5* (Paired box 5), *SARDH* (Sarcosine Dehydrogenase), *MAD1L1* (Mitotic Arrest Deficient 1 Like 1), *CUX1* (Cut Like Homeobox 1), *IFRD1* (Interferon Related Developmental Regulator 1), *PTPRN2* (Protein Tyrosine Phosphatase Receptor Type N2), *NOXA1* (NADPH Oxidase Activator 1), *FAM102A* ((Myc-DDK-tagged)-Human family with sequence similarity 102, member A), *NACC2* (NACC Family Member 2), *NUDT1* (Nudix Hydrolase 1), and *SNX8* (Sorting Nexin 8).

2. The composition for predicting or diagnosing metabolic syndrome according to claim 1,
wherein the CpG site is one or more CpG sites selected from the group consisting of chr9:6328440, chr9:36949608, chr9:136545908, chr9:136549044, chr7:1880699, chr7:1880700, chr7:1881369, chr7:1881370, chr7:1885069, chr7:1885070, chr7:1885114, chr7:1885115, chr7:1885119, chr7:1885221, chr7:1886937, chr7:1886938, chr7:1887361, chr7:1887362, chr7:1889520, chr7:1889521, chr7:1890925, chr7:1890926, chr7:1910374, chr7:1910450, chr7:1912056, chr7:1912057, chr7:1914757, chr7:1914758, chr7:1923695, chr7:1923696, chr7:1938655, chr7:1938656, chr7:1939765, chr7:1939766, chr7:1948712, chr7:1948713, chr7:1948755, chr7:1948756, chr7:1973579, chr7:1973580, chr7:1976457, chr7:1976862, chr7:1977906, chr7:1989946, chr7:2012996, chr7:2012997, chr7:2019875, chr7:2020994, chr7:2020995, chr7:2027323, chr7:2027324, chr7:2027354, chr7:2027355, chr7:2046830, chr7:2046831, chr7:2050401, chr7:2050402, chr7:2082536, chr7:2172066, chr7:2195443, chr7:101917140, chr7:157448562, chr7:157455211, chr7:157455296, chr9:140322640, chr9:130703789, chr9:138911484, chr7:2289889, chr7:2298227, chr7:2314651, chr7:2314652, chr7:220974, chr7:220975, chr7:223592, chr7:545084, chr7:1865921, chr7:1865922, chr7:1880737, chr7:1881265, chr7:1881266, chr7:1948948, chr7:1950615, chr7:1968227, chr7:2017445, chr7:2017446, chr7:2060127, chr7:2082524, chr7:2106607, chr7:2121196, and chr7:112087033.

3. The composition for predicting or diagnosing metabolic syndrome according to claim 1,
wherein the metabolic syndrome comprises one or more selected from the group consisting of hyperglycemia, central obesity, hypertension, and non-alcoholic fatty liver disease.

4. The composition for predicting or diagnosing metabolic syndrome according to claim 3,
wherein, when the metabolic syndrome comprises hyperglycemia,
the CpG site comprises one or more CpG sites selected from the group consisting of chr9:6328440, chr9:36949608, chr9:136545908, chr9:136549044, chr7:220974, chr7:220975, chr7:223592, and chr7:545084.

5. The composition for predicting or diagnosing metabolic syndrome according to claim 3,
wherein, when the metabolic syndrome comprises central obesity,
the CpG site comprises one or more CpG sites selected from the group consisting of chr7:1880699, chr7:1880700, chr7:1881369, chr7:1881370, chr7:1885069, chr7:1885070, chr7:1885114, chr7:1885115, chr7:1885119, chr7:1885221, chr7:1886937, chr7:1886938, chr7:1887361, chr7:1887362, chr7:1889520, chr7:1889521, chr7:1890925, chr7:1890926, chr7:1910374, chr7:1910450, chr7:1912056, chr7:1912057, chr7:1914757, chr7:1914758, chr7:1923695, chr7:1923696, chr7:1938655, chr7:1938656, chr7:1939765, chr7:1939766, chr7:1948712, chr7:1948713, chr7:1948755, chr7:1948756, chr7:1973579, chr7:1973580, chr7:1976457, chr7:1976862, chr7:1977906, chr7:1989946, chr7:2012996, chr7:2012997, chr7:2019875, chr7:2020994, chr7:2020995, chr7:2027323, chr7:2027324, chr7:2027354, chr7:2027355, chr7:2046830, chr7:2046831, chr7:2050401, chr7:2050402, chr7:2082536, chr7:2172066, chr7:2195443, chr7:101917140, chr7:157448562, chr7:157455211, chr7:157455296, chr9:140322640, chr9:130703789, chr9:138911484, chr7:1865921, chr7:1865922, chr7:1880737, chr7:1881265, chr7:1881266, chr7:1948948, chr7:1950615, chr7:1968227, chr7:2017445, chr7:2017446, chr7:2060127, chr7:2082524, chr7:2106607, chr7:2121196, and chr7:112087033.

6. The composition for predicting or diagnosing metabolic syndrome according to claim 3,
wherein, when the metabolic syndrome comprises hypertension,
the CpG site comprises chr7:2289889.

7. The composition for predicting or diagnosing metabolic syndrome according to claim 3,
wherein, when the metabolic syndrome comprises non-alcoholic fatty liver disease,
the CpG site comprises one or more CpG sites selected from the group consisting of chr7:2298227, chr7:2314651, and chr7:2314652.

8. The composition for predicting or diagnosing metabolic syndrome according to claim 1,
wherein the agent for measuring the methylation level of the CpG site of the gene comprises one or more selected from the group consisting of bisulfite or a salt thereof, a methylation-sensitive restriction enzyme, a primer specific for a methylated sequence at the CpG site of the promoter of the gene, a primer specific for an unmethylated sequence, a methylated CpG binding domain, and an antibody that specifically binds to methylcytosine.

9. The composition for predicting or diagnosing metabolic syndrome according to claim 8,
wherein the methylation-sensitive restriction enzyme comprises one or more selected from the group consisting of SmaI, SacII, EagI, HpaII, MspI, BssHII, BstUI, and NotI.

10. A kit for predicting or diagnosing metabolic syndrome, comprising the composition according to any one of claims 1 to 9.

11. A method for providing information for predicting or diagnosing metabolic syndrome, comprising a step of measuring the methylation level of a CpG site of one or more genes selected from the group consisting of *FAM20C* (Family with sequence similarity 20, member C), *PDGFA* (Platelet-derived growth factor subunit A), *TPD52L3* (Tumor Protein D52 Like 3), *PAX5* (Paired box 5), *SARDH* (Sarcosine Dehydrogenase), *MAD1L1* (Mitotic Arrest Deficient 1 Like 1), *CUX1* (Cut Like Homeobox 1), *IFRD1* (Interferon Related Developmental Regulator 1), *PTPRN2* (Protein Tyrosine Phosphatase Receptor Type N2), *NOXA1* (NADPH Oxidase Activator 1), *FAM102A* ((Myc-DDK-tagged)-Human family with sequence similarity 102, member A), *NACC2* (NACC Family Member 2), *NUDT1* (Nudix Hydrolase 1), and *SNX8* (Sorting Nexin 8) from a sample isolated from a subject.

12. The method for providing information for predicting or diagnosing metabolic syndrome according to claim 11,
wherein the CpG site is one or more CpG sites selected from the group consisting of chr9:6328440, chr9:36949608, chr9:136545908, chr9:136549044, chr7:1880699, chr7:1880700, chr7:1881369, chr7:1881370, chr7:1885069, chr7:1885070, chr7:1885114, chr7:1885115, chr7:1885119, chr7:1885221, chr7:1886937, chr7:1886938, chr7:1887361, chr7:1887362, chr7:1889520, chr7:1889521, chr7:1890925, chr7:1890926, chr7:1910374, chr7:1910450, chr7:1912056, chr7:1912057, chr7:1914757, chr7:1914758, chr7:1923695, chr7:1923696, chr7:1938655, chr7:1938656, chr7:1939765, chr7:1939766, chr7:1948712, chr7:1948713, chr7:1948755, chr7:1948756, chr7:1973579, chr7:1973580, chr7:1976457, chr7:1976862, chr7:1977906, chr7:1989946, chr7:2012996, chr7:2012997, chr7:2019875, chr7:2020994, chr7:2020995, chr7:2027323, chr7:2027324, chr7:2027354, chr7:2027355, chr7:2046830, chr7:2046831, chr7:2050401, chr7:2050402, chr7:2082536, chr7:2172066, chr7:2195443, chr7:101917140, chr7:157448562, chr7:157455211, chr7:157455296, chr9:140322640, chr9:130703789, chr9:138911484, chr7:2289889, chr7:2298227, chr7:2314651, chr7:2314652, chr7:220974, chr7:220975, chr7:223592, chr7:545084, chr7:1865921, chr7:1865922, chr7:1880737, chr7:1881265, chr7:1881266, chr7:1948948, chr7:1950615, chr7:1968227, chr7:2017445, chr7:2017446, chr7:2060127, chr7:2082524, chr7:2106607, chr7:2121196, and chr7:112087033.

13. The method for providing information for predicting or diagnosing metabolic syndrome according to claim 11,
wherein the metabolic syndrome comprises one or more selected from the group consisting of hyperglycemia, central obesity, hypertension, and non-alcoholic fatty liver disease.

14. The method for providing information for predicting or diagnosing metabolic syndrome according to claim 13,
wherein, when the metabolic syndrome comprises hyperglycemia,
the method comprises measuring the methylation level of a CpG site including one or more CpG sites selected from the group consisting of chr9:6328440, chr9:36949608, chr9:136545908, chr9:136549044, chr7:220974, chr7:220975, chr7:223592, and chr7:545084.

15. The method for providing information for predicting or diagnosing metabolic syndrome according to claim 13,
wherein, when the metabolic syndrome comprises central obesity,
the method comprises measuring the methylation level of a CpG site including one or more CpG sites selected from the group consisting of chr7:1880699, chr7:1880700, chr7:1881369, chr7:1881370, chr7:1885069, chr7:1885070, chr7:1885114, chr7:1885115, chr7:1885119, chr7:1885221, chr7:1886937, chr7:1886938, chr7:1887361, chr7:1887362, chr7:1889520, chr7:1889521, chr7:1890925, chr7:1890926, chr7:1910374, chr7:1910450, chr7:1912056, chr7:1912057, chr7:1914757, chr7:1914758, chr7:1923695, chr7:1923696, chr7:1938655, chr7:1938656, chr7:1939765, chr7:1939766, chr7:1948712, chr7:1948713, chr7:1948755, chr7:1948756, chr7:1973579, chr7:1973580, chr7:1976457, chr7:1976862, chr7:1977906, chr7:1989946, chr7:2012996, chr7:2012997, chr7:2019875, chr7:2020994, chr7:2020995, chr7:2027323, chr7:2027324, chr7:2027354, chr7:2027355, chr7:2046830, chr7:2046831, chr7:2050401, chr7:2050402, chr7:2082536, chr7:2172066, chr7:2195443, chr7:101917140, chr7:157448562, chr7:157455211, chr7:157455296, chr9:140322640, chr9:130703789, chr9:138911484, chr7:1865921, chr7:1865922, chr7:1880737, chr7:1881265, chr7:1881266, chr7:1948948, chr7:1950615, chr7:1968227, chr7:2017445, chr7:2017446, chr7:2060127, chr7:2082524, chr7:2106607, chr7:2121196, and chr7:112087033.

16. The method for providing information for predicting or diagnosing metabolic syndrome according to claim 13,
wherein, when the metabolic syndrome comprises hypertension,
the method comprises measuring the methylation level of a CpG site including chr7:2289889.

17. The method for providing information for predicting or diagnosing metabolic syndrome according to claim 13,
wherein, when the metabolic syndrome comprises non-alcoholic fatty liver disease,
the method comprises measuring the methylation level of a CpG site including one or more CpG sites selected from the group consisting of chr7:2298227, chr7:2314651, and chr7:2314652.

18. An apparatus for predicting or diagnosing metabolic syndrome, comprising:
a detection unit configured to measure a methylation level of a CpG site of one or more genes selected from the group consisting of *FAM20C* (Family with sequence similarity 20, member C), *PDGFA* (Platelet-derived growth factor subunit A), *TPD52L3* (Tumor Protein D52 Like 3), *PAX5* (Paired box 5), *SARDH* (Sarcosine Dehydrogenase), *MAD1L1* (Mitotic Arrest Deficient 1 Like 1), *CUX1* (Cut Like Homeobox 1), *IFRD1* (Interferon Related Developmental Regulator 1), *PTPRN2* (Protein Tyrosine Phosphatase Receptor Type N2), *NOXA1* (NADPH Oxidase Activator 1), *FAM102A* ((Myc-DDK-tagged)-Human family with sequence similarity 102, member A), *NACC2* (NACC Family Member 2), *NUDT1* (Nudix Hydrolase 1), and *SNX8* (Sorting Nexin 8) from a sample isolated from a subject; and
a determination unit configured to determine whether the CpG site is hypomethylated or hypermethylated.

19. The apparatus for predicting or diagnosing metabolic syndrome according to claim 18,
wherein the CpG site is one or more CpG sites selected from the group consisting of chr9:6328440, chr9:36949608, chr9:136545908, chr9:136549044, chr7:1880699, chr7:1880700, chr7:1881369, chr7:1881370, chr7:1885069, chr7:1885070, chr7:1885114, chr7:1885115, chr7:1885119, chr7:1885221, chr7:1886937, chr7:1886938, chr7:1887361, chr7:1887362, chr7:1889520, chr7:1889521, chr7:1890925, chr7:1890926, chr7:1910374, chr7:1910450, chr7:1912056, chr7:1912057, chr7:1914757, chr7:1914758, chr7:1923695, chr7:1923696, chr7:1938655, chr7:1938656, chr7:1939765, chr7:1939766, chr7:1948712, chr7:1948713, chr7:1948755, chr7:1948756, chr7:1973579, chr7:1973580, chr7:1976457, chr7:1976862, chr7:1977906, chr7:1989946, chr7:2012996, chr7:2012997, chr7:2019875, chr7:2020994, chr7:2020995, chr7:2027323, chr7:2027324, chr7:2027354, chr7:2027355, chr7:2046830, chr7:2046831, chr7:2050401, chr7:2050402, chr7:2082536, chr7:2172066, chr7:2195443, chr7:101917140, chr7:157448562, chr7:157455211, chr7:157455296, chr9:140322640, chr9:130703789, chr9:138911484, chr7:2289889, chr7:2298227, chr7:2314651, chr7:2314652, chr7:220974, chr7:220975, chr7:223592, chr7:545084, chr7:1865921, chr7:1865922, chr7:1880737, chr7:1881265, chr7:1881266, chr7:1948948, chr7:1950615, chr7:1968227, chr7:2017445, chr7:2017446, chr7:2060127, chr7:2082524, chr7:2106607, chr7:2121196, and chr7:112087033.

20. The apparatus for predicting or diagnosing metabolic syndrome according to claim 18,
wherein the metabolic syndrome comprises one or more selected from the group consisting of hyperglycemia, central obesity, hypertension, and non-alcoholic fatty liver disease.

21. A method for predicting or diagnosing metabolic syndrome, comprising a step of measuring a methylation level of a CpG site of one or more genes selected from the group consisting of *FAM20C* (Family with sequence similarity 20, member C), *PDGFA* (Platelet-derived growth factor subunit A), *TPD52L3* (Tumor Protein D52 Like 3), *PAX5* (Paired box 5), *SARDH* (Sarcosine Dehydrogenase), *MAD1L1* (Mitotic Arrest Deficient 1 Like 1), *CUX1* (Cut Like Homeobox 1), *IFRD1* (Interferon Related Developmental Regulator 1), *PTPRN2* (Protein Tyrosine Phosphatase Receptor Type N2), *NOXA1* (NADPH Oxidase Activator 1), *FAM102A* ((Myc-DDK-tagged)-Human family with sequence similarity 102, member A), *NACC2* (NACC Family Member 2), *NUDT1* (Nudix Hydrolase 1), and *SNX8* (Sorting Nexin 8) from a sample isolated from a subject suspected of having metabolic syndrome.

22. The method for predicting or diagnosing metabolic syndrome according to claim 21,
wherein the CpG site is one or more CpG sites selected from the group consisting of chr9:6328440, chr9:36949608, chr9:136545908, chr9:136549044, chr7:1880699, chr7:1880700, chr7:1881369, chr7:1881370, chr7:1885069, chr7:1885070, chr7:1885114, chr7:1885115, chr7:1885119, chr7:1885221, chr7:1886937, chr7:1886938, chr7:1887361, chr7:1887362, chr7:1889520, chr7:1889521, chr7:1890925, chr7:1890926, chr7:1910374, chr7:1910450, chr7:1912056, chr7:1912057, chr7:1914757, chr7:1914758, chr7:1923695, chr7:1923696, chr7:1938655, chr7:1938656, chr7:1939765, chr7:1939766, chr7:1948712, chr7:1948713, chr7:1948755, chr7:1948756, chr7:1973579, chr7:1973580, chr7:1976457, chr7:1976862, chr7:1977906, chr7:1989946, chr7:2012996, chr7:2012997, chr7:2019875, chr7:2020994, chr7:2020995, chr7:2027323, chr7:2027324, chr7:2027354, chr7:2027355, chr7:2046830, chr7:2046831, chr7:2050401, chr7:2050402, chr7:2082536, chr7:2172066, chr7:2195443, chr7:101917140, chr7:157448562, chr7:157455211, chr7:157455296, chr9:140322640, chr9:130703789, chr9:138911484, chr7:2289889, chr7:2298227, chr7:2314651, chr7:2314652, chr7:220974, chr7:220975, chr7:223592, chr7:545084, chr7:1865921, chr7:1865922, chr7:1880737, chr7:1881265, chr7:1881266, chr7:1948948, chr7:1950615, chr7:1968227, chr7:2017445, chr7:2017446, chr7:2060127, chr7:2082524, chr7:2106607, chr7:2121196, and chr7:112087033.

23. The method for predicting or diagnosing metabolic syndrome according to claim 21,
wherein the metabolic syndrome comprises one or more selected from the group consisting of hyperglycemia, central obesity, hypertension, and non-alcoholic fatty liver disease.

24. Use of an agent capable of measuring a methylation level of a CpG site of one or more genes selected from the group consisting of *FAM20C* (Family with sequence similarity 20, member C), *PDGFA* (Platelet-derived growth factor subunit A), *TPD52L3* (Tumor Protein D52 Like 3), *PAX5* (Paired box 5), *SARDH* (Sarcosine Dehydrogenase), *MAD1L1* (Mitotic Arrest Deficient 1 Like 1), *CUX1* (Cut Like Homeobox 1), *IFRD1* (Interferon Related Developmental Regulator 1), *PTPRN2* (Protein Tyrosine Phosphatase Receptor Type N2), *NOXA1* (NADPH Oxidase Activator 1), *FAM102A* ((Myc-DDK-tagged)-Human family with sequence similarity 102, member A), *NACC2* (NACC Family Member 2), *NUDT1* (Nudix Hydrolase 1), and *SNX8* (Sorting Nexin 8) for predicting or diagnosing metabolic syndrome.

25. The use for predicting or diagnosing metabolic syndrome according to claim 24,
wherein the agent is capable of measuring a methylation level of one or more CpG sites selected from the group consisting of chr9:6328440, chr9:36949608, chr9:136545908, chr9:136549044, chr7:1880699, chr7:1880700, chr7:1881369, chr7:1881370, chr7:1885069, chr7:1885070, chr7:1885114, chr7:1885115, chr7:1885119, chr7:1885221, chr7:1886937, chr7:1886938, chr7:1887361, chr7:1887362, chr7:1889520, chr7:1889521, chr7:1890925, chr7:1890926, chr7:1910374, chr7:1910450, chr7:1912056, chr7:1912057, chr7:1914757, chr7:1914758, chr7:1923695, chr7:1923696, chr7:1938655, chr7:1938656, chr7:1939765, chr7:1939766, chr7:1948712, chr7:1948713, chr7:1948755, chr7:1948756, chr7:1973579, chr7:1973580, chr7:1976457, chr7:1976862, chr7:1977906, chr7:1989946, chr7:2012996, chr7:2012997, chr7:2019875, chr7:2020994, chr7:2020995, chr7:2027323, chr7:2027324, chr7:2027354, chr7:2027355, chr7:2046830, chr7:2046831, chr7:2050401, chr7:2050402, chr7:2082536, chr7:2172066, chr7:2195443, chr7:101917140, chr7:157448562, chr7:157455211, chr7:157455296, chr9:140322640, chr9:130703789, chr9:138911484, chr7:2289889, chr7:2298227, chr7:2314651, chr7:2314652, chr7:220974, chr7:220975, chr7:223592, chr7:545084, chr7:1865921, chr7:1865922, chr7:1880737, chr7:1881265, chr7:1881266, chr7:1948948, chr7:1950615, chr7:1968227, chr7:2017445, chr7:2017446, chr7:2060127, chr7:2082524, chr7:2106607, chr7:2121196, and chr7:112087033.

26. The use for predicting or diagnosing metabolic syndrome according to claim 24,
wherein the metabolic syndrome comprises one or more selected from the group consisting of hyperglycemia, central obesity, hypertension, and non-alcoholic fatty liver disease.
